# EUROPÄISCHE PATENTSCHRIFT

(11) **EP 0 537 592 B1**
(45) Veröffentlichungstag und Bekanntmachung des Hinweises auf die Patenterteilung: **17.01.1996**
(21) Anmeldenummer: 92117009.8
(22) Anmeldetag: 06.10.1992
(51) Int. Cl.: C07C 205/26

(54) **Verfahren zur Herstellung von 2-Nitro-5-fluor- bzw. -5-chlor-phenol**
Process for the preparation of 2-nitro-5-fluoro- respectively -5-chloro-phenol
Procédé pour la préparation de 2-nitro-5-fluoro- respectivement -5-chloro-phénol

(30) Priorität: 09.10.1991 DE 4133447
(43) Veröffentlichungstag der Anmeldung: 21.04.1993
(73) Patentinhaber: HOECHST AKTIENGESELLSCHAFT, D-65926 Frankfurt am Main (DE)
(72) Erfinder: Pfirmann, Ralf, Dr., W-6103 Griesheim (DE); Papenfuhs, Theodor, Dr., W-6000 Frankfurt/Main (DE); Forstinger, Klaus, Dr., W-6092 Kelsterbach (DE)

(56) Entgegenhaltungen:
- EP-A- 0 000 542
- WO-A-92/05141
- GB-A- 2 058 068
- US-A- 4 596 893
- CHEMICAL ABSTRACTS, vol. 110, 1989, Seite 708, Spalte 2, Zusammenfassung Nr. 192429f, Columbus, Ohio, US; T. HAGA et al: "Preparation of 5-fluoro-2-nitrophenol as intermediate for herbicides"

## Beschreibung

Die vorliegende Erfindung betrifft ein verbessertes Verfahren zur Herstellung von 2-Nitro-5-fluor- bzw. -5-chlor-phenol in guten Ausbeuten und hoher Selektivität. Die Verbindungen stellen wertvolle Vorprodukte für die Herstellung von Pflanzenschutz- und Arzneimitteln dar.

Es ist bekannt, daß die genannte Fluorverbindung bei langen Reaktionszeiten (32 Stunden) in Ethern, wie Dioxan, unter Phasentransferkatalyse bei niedrigen Temperaturen (30°C) hergestellt werden kann (JP 63310851 A2). Arbeiten in Wasser ergibt hierbei ein Isomerenverhältnis von etwa 7 zu 1; über die Trennung der Isomeren ist aus dieser Quelle nichts bekannt. Es ist ferner bekannt, die Chlorverbindung ebenfalls in Lösungsmittel-Wasser-Gemischen in Ausbeuten um 90 % herstellen zu können (DE 29 39 056). Bei einem älteren bekannten Verfahren (DE 29 35 629) werden wäßrige Alkalilaugen in polar aprotischen Losungsmitteln und Emulgatoren (20 Stunden, 60°C, 81 %) für diese Reaktion verwendet. Schließlich ist bekannt, daß sich allgemein 2-Nitrohalophenole durch Reaktion der entsprechenden Nitrohalogenbenzole bei hohen Temperaturen (155°C, 2 Stunden) in wasserfreien Medien herstellen lassen. Auf diese Weise erhält man 4,5-Dichlor-2-nitrophenol in 77 % Ausbeute (DE 26 14 264). Durch Umsetzung von 4-Chlornitrobenzol mit KOH bei 50°C in Anwesenheit von Glaskörpern (FR 1 581 400) erhält man 2-Nitro-5-chlorphenol. Dieselbe Oxidation kann in Lösungsmitteln, wie beispielsweise 1,2-Dimethoxyethan unter Phasentransferkatalyse (18-Krone-6) in 85 - 87 % Ausbeute durchgeführt werden (Izv. Akad. Nauk. SSSR, Ser. Khim., (11), 2635-2636). In tertiären Alkoholen erhält man Ausbeuten um 90 % bei Anwendung von wäßriger Kalilauge (6 Stunden, 60°C) (JP 52142031). Schon lange bekannt ist die Nitrierung von 3-Chlorphenol (Uhlemann, Chem. Ber. 11 (1878), 1161; De Kiewiet, Stephen, J. Chem. Soc. (1931), 84). Diese Nitrierung kann ebenfalls in Essigsäure bei 0°C (Roberts, Rhys, J.Chem. Soc. (1937), 39, 41) oder mit Natriumnitrat und verdünnter Schwefelsäure (Hodgson, Moore, J.Chem. Soc. 127 (1925), 1600) durchgeführt werden. Eine Variante ist die Umsetzung von 3-Chloranilin mit verdünnter Salpetersäure (Uhlemann, loc. cit.) oder nitrosen Gasen (NOₓ), wobei bei letzterer allerdings neben dem gewünschten Produkt Isomere entstehen (v. Auwers, Deines, Fortschr. Ch. Phys. 18, 59; Chem. Zentralblatt, 1924 (II), 2268). Des weiteren sind Umsetzungen von 4-Chlor-1,2-dinitrobenzol mit wäßriger Alkalilauge (Laubenheimer, Chem. Ber. 9 (1876), 768) und von 2-Nitro-5-amino-phenol im Sinne einer Sandmeyer-Reaktion mit Kupfer(I)-chlorid bekannt (Philips, J. Chem. Soc. (1930), 1910, 1913), wobei man zu 2-Nitro-5-chlorphenol gelangt.

Da 2-Nitro-5-fluor- bzw. -5-chlor-phenol nach literaturbekannten Methoden zu Herbiziden (EP 304409 A1, JP 63310851 A2, US 4 734 124), Insektiziden (DuPont de Nemours, E.I. & Co., WO 9003378 A1), Haarfärbemitteln (DE 35 33 792 A1), pharmakologisch wirksamen Substanzen, wie Lipoxygenaseinhibitoren (EP 262 618 A2) oder Blutdruckregulatoren (DE 35 24 635 A1) oder antibakteriell wirksamen Substanzen (US 3 461 173, US 3 551 503) umgesetzt werden können und außerdem schon für die inredestehenden Verbindungen fungizide Wirksamkeit festgestellt werden konnte (R.H. Shiley, J. L. Forsberg, R.S. Perry, D.R. Dickerson, G.C. Finger, J. Fluorine Chem., 5(4), 371-376), bestand ein Bedürfnis, diese Verbindungen nach einem neuen, verbesserten, möglichst einfachen und ökonomischen Verfahren herstellen zu können.

Es wurde nun gefunden, daß man isomerenreines 2-Nitro-5-fluor- bzw. -5-chlorphenol in guten bis sehr guten Ausbeuten und guter Selektivität vorteilhaft herstellen kann, indem 2,4-Difluornitrobenzol bzw. 2,4-Dichlornitrobenzol mit wäßriger Alkalimetall- oder Erdalkalimetallhydroxidlösung oder -Suspension, vorzugsweise Natriumhydroxid- oder Kaliumhydroxidlösung, in Abwesenheit organischer Lösungsmittel oder sonstiger löslichkeitsvermittelnder Substanzen bei Temperaturen von 20°C bis 190°C, vorzugsweise von 20°C bis 70°C im Falle des 2,4-Difluornitrobenzols, und von 100°C bis 160°C im Falle des 2,4-Dichlornitrobenzols, umsetzt, das Reaktionsgemisch durch Zugabe von Säure auf einen pH-Wert von 1 bis 6, vorzugsweise von 1,5 bis 3,0, stellt, das entstandene Produkt durch Einleiten von Wasserdampf in die Reaktionsmischung wasserdampfdestilliert und nach dem Abkühlen aus dem Destillat isoliert.

Bei keinem Schritt des erfindungsgemäßen Verfahrens ist ein organisches Lösungsmittel zugegen.

Im Zuge der Verfahrensdurchführung im einzelnen ist es auch möglich, das nach dem Ansäuern ausgefallene Produkt abzusaugen und erst danach der Wasserdampfdestillation zu unterwerfen. Dies hat im Falle der Fluorverbindung den Vorteil, daß die Fluorwasserstoffsaure vor Einleitung des Wasserdampfes abgetrennt werden kann. Andernfalls kann durch Zusatz von fluoridfangenden Mitteln die Korrosion an den verwendeten Apparaturen gering gehalten werden. In Frage für diesen Zweck kommen Calciumsalze, wie beispielsweise Calciumchlorid, Calciumsulfat, Calciumhydroxid oder Siliciumdioxid, besonders bevorzugt Siliciumdioxid mit erhöhter innerer Oberfläche, oder Tributylzinnverbindungen, wie beispielsweise Tri-n-butylzinnchlorid. Diese fluoridfangenden Mittel werden in der etwa 0,5 bis etwa 10-fachen, bevorzugt in der etwa 1,5- bis etwa 4-fachen der theoretisch entstandenen Fluoridmenge eingesetzt. Es war überraschend, daß sich auch in der bei dem erfindungsgemäßen Verfahren entstehenden verdünnten wäßrigen Lösung die Korrosion durch Fluorwasserstoffsäure weitgehend unterdrücken ließ.

Das erfindungsgemäße Verfahren kann im einzelnen auch so durchgeführt werden, daß das Ansäuern allmählich während der Wasserdampfdestillation erfolgt. Dadurch werden die Apparatematerialien besonders geschont und das Produkt kann dennoch in guten bis sehr guten Ausbeuten isoliert werden.

Um eine hohe Selektivität zu erhalten und die Rührbarkeit der Lösung während der Reaktion zu gewährleisten, wird zweckmäßigerweise die Alkali- oder Erdalkalimetallhydroxidlösung zu einer vorgelegten Mischung aus 2,4-Difluornitrobenzol oder 2,4-Dichlornitrobenzol zudosiert. Bei der Chlorverbindung kann auf diese Vorgehensweise verzichtet werden. Man erhält denselben Effekt, wenn man die Reaktionspartner vorlegt und die Reaktionsmischung langsam hochheizt.

Das erfindungsgemäße Verfahren liefert das 2-Nitro-5-fluorphenol in isomerenreiner Form in 78 %iger Ausbeute, während das 2-Nitro-5-chlorphenol in 91 %iger Ausbeute erhalten werden kann. Das Verfahren wird zweckmäßigerweise bei Normaldruck durchgeführt; die Umsetzung kann jedoch auch bei Überdruck vorgenommen werden, wie es sich bei der Umsetzung von 2,4-Dichlornitrobenzol gemäß dem erfindungsgemäßen Verfahren als notwendig erweist, um eine genügend schnelle Umsetzung zu erhalten.

Als wäßrige Alkalimetallhydroxidlösungen können beispielsweise Lithium-, Natrium-, Kalium-, Rubidium- oder Cäsiumhydroxidlösungen oder Mischungen daraus, vorzugsweise Natrium- oder Kaliumhydroxidlösungen, als Erdalkalimetallhydroxidlösungen beispielsweise Magnesium-, Calcium-, Strontium- oder Bariumhydroxidlösungen oder Mischungen daraus, vorzugsweise Calciumhydroxidlösungen, angewandt werden. Die genannten Hydroxide werden in Form etwa 10- bis etwa 80 %iger Lösungen, vorzugsweise etwa 30- bis etwa 50 %iger Lösungen eingesetzt. Ebenso zweckmäßig ist es, anstatt der vorstehend genannten Lösungen Suspensionen der entsprechenden Alkali- oder Erdalkalimetallhydroxide zu verwenden, sofern diese sich nicht in der jeweilig verwendeten Wassermenge zu lösen vermögen.

Das Ansäuern kann mit den üblichen nicht-oxidierenden Mineralsäuren, wie beispielsweise verdünnter Schwefelsäure, Phosphorsäure, Salzsäure, Bromwasserstoffsäure oder Jodwasserstoffsäure, sowie mit ausreichend starken organischen Säuren, wie beispielsweise Ameisensäure oder Essigsäure, erfolgen.

Durch die nachstehenden Beispiele wird das erfindungsgemäße Verfahren näher erläutert, ohne darauf beschränkt zu werden.

### Beispiel 1

159,1 g (1 Mol) 2,4-Difluornitrobenzol und 550 g Wasser werden vorgelegt und auf 55°C geheizt. Unter kräftigem Rühren tropft man binnen 4 Stunden 241,2 g (2,15 Mol) 50 %ige Kaliumhydroxidlösung zu und hält dabei die Temperatur auf 55°C (exotherme Reaktion). Bei dieser Temperatur wird noch 2 Stunden nachgerührt. Anschließend stellt man mit 88 g Schwefelsäure auf pH 4,3 ein (25°C) und gibt 74,1 g Calciumhydroxid zu. Man stellt den pH-Wert erneut auf 5,0 ein und beginnt, Wasserdampf einzuleiten. Während der Destillation senkt man durch Zutropfen von Schwefelsäure den pH-Wert von 5 auf 1,5 ab. Das Produkt wird durch Abkühlen des Destillates und Filtration isoliert. Man erhält nach dem Trocknen 117,9 g (0,750 Mol, 75 % d. Th.) leuchtendgelbes 2-Nitro-5-fluorphenol, das nach GC und HPLC einen Reingehalt von über 99,9 % besitzt (Ep. 32,1°C).
Setzt man anstatt Kaliumhydroxid Natriumhydroxid ein und/oder säuert man statt mit Schwefelsäure mit Salzsäure an und/oder verwendet man statt Calciumhydroxid Calciumchlorid als fluoridfangendes Mittel, so erhält man im wesentlichen dasselbe Ergebnis.

### Beispiel 2

Man legt 159,1 g (1 Mol) 2,4-Difluornitrobenzol und 300 g Wasser vor und tropft bei 45°C 52,7 g (2,2 Mol) 50 %ige Lithiumhydroxidlösung zu. Durch die exotherme Reaktion läßt man die Temperatur auf 55°C steigen. Nach Ende der Dosierung (5 Stunden) gibt man 60,1 g Siliciumdioxid (®Aerosil) zu und stellt den pH-Wert mit Schwefelsäure auf 1,5 ein. Durch Einleiten von Wasserdampf wird das Produkt überdestilliert und, wie in Beispiel 1 beschrieben, isoliert. Es werden 115,5 g (0,735 Mol, 74 % d.Th.) 2-Nitro-5-fluorphenol erhalten, die sich im Reingehalt nicht von dem nach Beispiel 1 hergestellten Material unterscheiden.
Anstatt Lithiumhydroxid kann unter Erhalt desselben Ergebnisses Lithiumcarbonat verwendet werden. Verwendet man statt Schwefelsäure Phosphorsäure zum Sauerstellen der Reaktionsmischung, so erhält man im wesentlichen dasselbe Ergebnis.

### Beispiel 3

Zu 159,1 g (1 Mol) 2,4-Difluornitrobenzol tropft man bei 40°C in 6 Stunden 749,6 g (2,12 Mol) 20 %ige Cäsiumhydroxidlösung und rührt danach 1 Stunde nach. Man stellt mit 85 %iger Phosphorsäure auf pH 2 und destilliert das Produkt mit Wasserdampf über (1,5 Stunden). Nach Aufarbeitung, wie in Beispiel 1 beschrieben, erhält man 120,2 g (0,765 Mol, 77 % d. Th.) 2-Nitro-5-fluorphenol (RG(GC) > 99,9 %).
Verwendet man zum Ansäuern Bromwasserstoffsäure anstatt Phosphorsäure, so gelangt man im wesentlichen zum selben Ergebnis.

### Beispiel 4

192,0 g (1 Mol) 2,4-Dichlornitrobenzol, 600 g Wasser und 273,0 g (2,05 Mol) Natriumhydroxid werden in einem VA-Autoklaven unter guter Rührung 6 Stunden auf 140°C geheizt. Die ausreagierte Reaktionsmischung wird bei 70°C dem Autoklaven entnommen. Die anfallende orange-braune Lösung wird mit 30 %iger Salzsäure auf pH 2 eingestellt und das Produkt mit Wasserdampf überdestilliert. Aus dem Destillat (etwa 1,5 l) erhält man durch Ausführen bei 10°C 2-Nitro-5-chlorphenol (156,4 g, 90 % d. Th.) in Form gelber Kristalle.
Säuert man statt mit 30 %iger Salzsäure mit Ameisensäure auf pH 2, so erhält man im wesentlichen dasselbe Ergebnis. Anstelle von Natriumhydroxid kann in gleicher Menge Natriumcarbonat ohne wesentliche Änderungen im Resultat verwendet werden.

### Beispiel 5

192,0 g (1 Mol) 2,4-Dichlornitrobenzol, 750 g Wasser und 185,3 g (2,5 Mol) Calciumhydroxid werden unter Druck 14 Stunden auf 150°C erhitzt. Anschließend filtriert man bei 70°C die ungelösten Bestandteile ab. Das Filtrat wird mit Jodwasserstoffsäure auf pH 1 gestellt und das Produkt mit Wasserdampf überdestilliert. Durch Ausrühren des Destillates erhält man 2-Nitro-5-chlorphenol (151,0 g, 87 % d.Th.), das einen Reingehalt von über 99,9 % (GC, HPLC) aufweist.

Säuert man statt mit Jodwasserstoffsäure mit Essigsäure auf pH 2,5 an, so erhält man im wesentlichen dasselbe Ergebnis. Anstelle von Calciumhydroxid kann in gleicher Menge Calciumcarbonat ohne wesentliche Änderungen im Resultat verwendet werden.

## Patentansprüche

1. Verfahren zur Herstellung von isomerenreinem 2-Nitro-5-fluor- bzw. -5-chlorphenol, dadurch gekennzeichnet, daß man 2,4-Difluornitrobenzol bzw. 2,4-Dichlornitrobenzol mit wäßriger Alkalimetall- oder Erdalkalimetallhydroxidlösung oder -suspension in Abwesenheit organischer Lösungsmittel oder sonstiger löslichkeitsvermittelnder Substanzen bei Temperaturen von 20°C bis 190°C umsetzt, das Reaktionsgemisch durch Zugabe von Säure auf einen pH-Wert von 1 bis 6 stellt, das entstandene Produkt wasserdampfdestilliert und nach dem Abkühlen aus dem Destillat isoliert.

2. Verfahren nach Anspruch 1, dadurch gekennzeichnet, daß man 2,4-Difluornitrobenzol bei Temperaturen von 40°C bis 70°C umsetzt.

3. Verfahren nach Anspruch 1, dadurch gekennzeichnet, daß man 2,4-Dichlornitrobenzol bei Temperaturen von 100°C bis 160°C umsetzt.

4. Verfahren nach mindestens einem der Ansprüche 1 bis 3, dadurch gekennzeichnet, daß man das Reaktionsgemisch auf einen pH-Wert von 1,5 bis 3,0 einstellt.

5. Verfahren nach mindestens einem der Ansprüche 1 bis 4, dadurch gekennzeichnet, daß man als wäßrige Alkalimetallhydroxidlösung wäßrige Lithium-, Natrium-, Kalium-, Rubidium- oder Cäsiumhydroxidlösung oder Mischungen daraus verwendet.

6. Verfahren nach mindestens einem der Ansprüche 1 bis 5, dadurch gekennzeichnet, daß man als wäßrige Erdalkalimetallhydroxidlösung wäßrige Magnesium-, Calcium-, Strontium- oder Bariumhydroxidlösung oder Mischungen daraus verwendet.

7. Verfahren nach mindestens einem der Ansprüche 1 bis 6, dadurch gekennzeichnet, daß man mit einer nichtoxidierenden Mineralsäure ansäuert.

8. Verfahren nach mindestens einem der Ansprüche 1 bis 7, dadurch gekennzeichnet, daß das Ansäuern der Reaktionsmischung während der Wasserdampfdestillation parallel zum Austrag des Produktes erfolgt.

9. Verfahren nach mindestens einem der Ansprüche 1 bis 8, dadurch gekennzeichnet, daß man vor dem Ansäuern der ausreagierten Reaktionsmischung fluoridabfangende Mittel zusetzt.

10. Verfahren nach mindestens einem der Ansprüche 1 bis 9, dadurch gekennzeichnet, daß man vor dem Ansäuern der ausreagierten Reaktionsmischung als fluoridabfangende Mittel Calciumchlorid, -sulfat oder -hydroxid oder Siliciumdioxid oder Tributylzinnverbindungen verwendet.

11. Verfahren nach mindestens einem der Ansprüche 1 bis 10, dadurch gekennzeichnet, daß man das Verfahren bei Atmosphären- oder Überdruck durchführt.

## Claims

1. A process for the preparation of isomerically pure 2-nitro-5-fluoro- or -5-chlorophenol, which comprises reacting 2,4-difluoronitrobenzene or 2,4-dichloronitrobenzene with aqueous alkali metal or alkaline earth metal hydroxide solution or suspension in the absence of organic solvents or other solubilizers at temperatures from 20°C to 190°C, adjusting the pH of the reaction mixture to 1 to 6 by the addition of acid, steam distilling the resultant product and isolating it from the distillate after cooling.

2. The process as claimed in claim 1, wherein 2,4-difluoronitrobenzene is reacted at temperatures from 40°C to 70°C.

3. The process as claimed in claim 1, wherein 2,4-dichloronitrobenzene is reacted at temperatures from 100°C to 160°C.

4. The process as claimed in at least one of claims 1 to 3, wherein the pH of the reaction mixture is adjusted to 1.5 to 3.0.

5. The process as claimed in at least one of claims 1 to 4, wherein the aqueous alkali metal hydroxide solution used is aqueous lithium, sodium, potassium, rubidium or cesium hydroxide solution or mixtures thereof.

6. The process as claimed in at least one of claims 1 to 5, wherein the aqueous alkaline earth metal hydroxide solution used is aqueous magnesium, calcium, strontium or barium hydroxide solution or mixtures thereof.

7. The process as claimed in at least one of claims 1 to 6, wherein acidification takes place using a non-oxidizing mineral acid.

8. The process as claimed in at least one of claims 1 to 7, wherein acidification of the reaction mixture takes place during steam distillation in parallel with discharge of the product.

9. The process as claimed in at least one of claims 1 to 8, wherein fluoride scavengers are added to the reacted reaction mixture before acidification.

10. The process as claimed in at least one of claims 1 to 9, wherein the fluoride scavengers used before acidifying the reacted reaction mixture are calcium chloride, calcium sulfate, calcium hydroxide or silicon dioxide or tributyltin compounds.

11. The process as claimed in at least one of claims 1 to 10, wherein the process is performed at atmospheric or excess pressure.

## Revendications

1. Procédé de préparation de 2-nitro-5-fluoro- ou -5-chlorophénol sous forme d'isomères purs, caractérisé en ce que l'on fait réagir respectivement du 2,4-difluoronitrobenzène ou du 2,4-dichloronitrobenzène avec une solution ou une suspension aqueuse d'hydroxyde de métal alcalin ou de métal alcalino-terreux, en l'absence de solvant organique ou d'autres substances de solubilisation, à des températures comprises entre 20°C et 190°C, on amène le mélange réactionnel à un pH de 1 à 6 en ajoutant un acide, on soumet le produit obtenu à un entraînement à la vapeur, et on l'isole à partir du distillat après refroidissement.

2. Procédé selon la revendication 1, caractérisé en ce que l'on fait réagir du 2,4-difluoronitrobenzène à des températures comprises entre 40°C et 70°C.

3. Procédé selon la revendication 1, caractérisé en ce que l'on fait réagir du 2,4-dichloronitrobenzène à des températures comprises entre 100°C et 160°C.

4. Procédé selon l'une au moins des revendications 1 à 3, caractérisé en ce que l'on amène le mélange réactionnel à un pH de 1,5 à 3,0.

5. Procédé selon l'une au moins des revendications 1 à 4, caractérisé en ce que l'on utilise comme solution aqueuse d'hydroxyde de métal alcalin une solution aqueuse d'hydroxyde de lithium, de sodium, de potassium, de rubidium ou de césium ou un de leurs mélanges.

6. Procédé selon l'une au moins des revendications 1 à 5, caractérisé en ce que l'on utilise comme solution aqueuse d'hydroxyde de métal alcalino-terreux une solution aqueuse d'hydroxyde de magnésium, de calcium, de strontium ou de baryum ou un de leurs mélanges.

7. Procédé selon l'une au moins des revendications 1 à 6, caractérisé en ce que l'on acidifie avec un acide minéral non oxydant.

8. Procédé selon l'une au moins des revendications 1 à 7, caractérisé en ce que l'acidification du mélange réactionnel s'effectue pendant l'entraînement à la vapeur parallèlement à la séparation du produit.

9. Procédé selon l'une au moins des revendications 1 à 8, caractérisé en ce que, avant d'acidifier le mélange ayant cessé de réagir, on ajoute un agent fixateur de fluorures.

10. Procédé selon l'une au moins des revendications 1 à 9, caractérisé en ce que, avant d'acidifier le mélange réactionnel ayant cessé de réagir, on utilise comme agent fixateur de fluorures du chlorure, du sulfate ou de l'hydroxyde de calcium ou du dioxyde de silicium ou des composés de tributylétain.

11. Procédé selon l'une au moins des revendications 1 à 10, caractérisé en ce que l'on effectue le procédé sous la pression atmosphérique ou sous une surpression.
